(19) Europäisches Patentamt
European Patent Office
Office européen des brevets

(11) Publication number: 0 026 561 A2

(12) # EUROPEAN PATENT APPLICATION

(21) Application number: 80302491.8

(22) Date of filing: 23.07.80

(51) Int. Cl.[3]: **A 61 K 37/02**
A 61 K 35/24, A 61 K 35/42
A 61 K 35/407, A 61 K 35/28
A 61 K 35/34, A 61 K 35/22
C 07 H 13/04

(30) Priority: 26.09.79 JP 123669/79

(43) Date of publication of application: 08.04.81 Bulletin 81/14

(84) Designated Contracting States: AT BE CH DE FR GB IT LI LU NL SE

(71) Applicant: Shibata, Seiichi
40-11, Takatanobaba 4-chome Shinjuku-ku
Tokyo(JP)

(72) Inventor: Shibata, Seiichi
40-11, Takatanobaba 4-chome Shinjuku-ku
Tokyo(JP)

(74) Representative: Dew, Melvyn John et al,
Haseltine Lake & Co Hazlitt House 28 Southampton Buildings Chancery Lane
London WC2A 1AT(GB)

(54) **Nephritogenic compounds containing a biologically active sugar moiety, methods of preparing them and their applications.**

(57) A novel nephrotogenic compound has a biologically active sugar moiety of structural formula

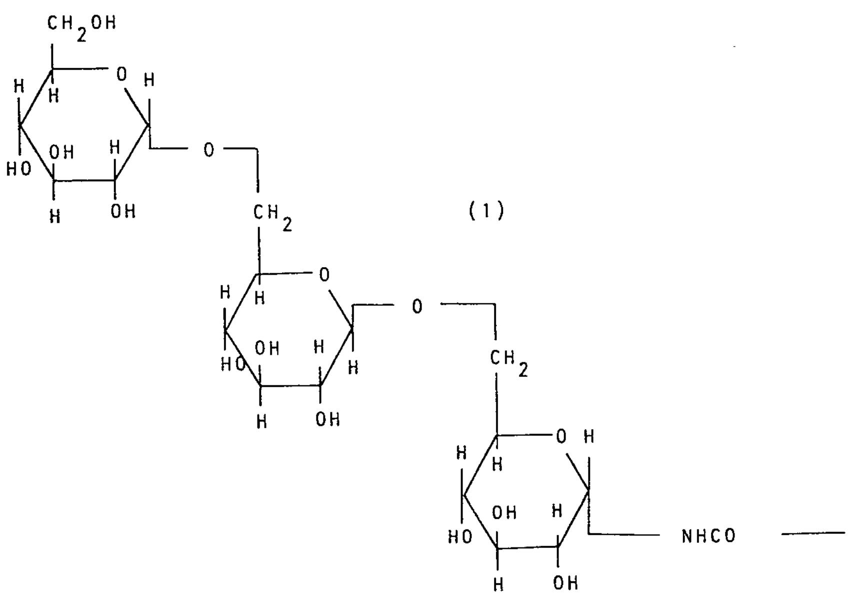

(1)

./...

EP 0 026 561 A2

The compound may be a glycopeptide which contains a peptide moiety linking to the sugar moiety through the N-glycoside bond. The glycopeptide may be isolated from the organs of normal animals, especially the kidneys, or from the urine of healthy human beings, and is biological active to induce kidney lesions in animals. The lesions induced by an injection of the glycopeptide are very similar to the spectrum of human glomerulonephritis, and so the injected animals provide an experimental model for studies on human glomerulonephritis.

TITLE MODIFIED
see front page



Nephrotogenic compounds containing a biologically active sugar moiety.

This invention relates to novel compounds containing a specific biologically active sugar moiety, and is particularly but not exclusively concerned with novel glycopeptides in which a peptide moiety is linked to the sugar moiety through the N-glycoside bond and processes of isolating them. The invention also relates to an agent for inducing glomerulonephritis in animals and to a method of preparing an experimental model for the study of human glomerulonephritis.

In the medical and pharmaceutical fields, especially in the development of therapeutic treatments of human diseases and the screening of medicines, it is important to be able to prepare stable experimental models plentifully and economically. By experimental model there is meant an experimental animal having lesions which are strikingly similar to the spectrum of the human disease. In recent times the supply of experimental models such as rats with hypertension, mice with diabetes and rats with digestive ulcers has contributed remarkably to the development of therapeutic treatments for humans.

With regard to human glomerulonephritis, many experimental models have been reported; however most are

concerned with acute glomerulonephritis and so have only a similarity to human glomerulonephritis in terms of morphologic changes: the models have no similarity in terms of clinical functions such as metabolism. Thus the experimental models which are known cannot be used for the developments of diagnosis and therapeutic agents.

One well known experimental model is Masugi's glomerulonephritis, in which glomerulonephritis is induced, together with proteinuria, by the injection of a particular serum. This serum is obtainable by the steps of (1) injecting a homogenate of kidney excised from a normal animal into a heterologous animal once or twice a week for about 2 months; (2) collecting blood from the animal; and (3) separating the serum from the blood. To induce the condition the serum is injected into a normal animal which is homologous to the animal from which kidney is excised. As is apparent from the procedure adopted, it is believed that Masugi's glomerulonephritis is induced by means of an antigen-antibody reaction using the homogenate of kidney.

Many subsequent attempts have been made to purify the homogenate in order to isolate a complete antigen substance that yields "nephrotoxic antiserum" having an ability to induce glomerulonephritis. Anatomical studies have shown that the complete antigen substance is mainly present in glomerulus of kidney, especially glomerular basement membrane (GBM). Although many attempts have been made, a complete chemical analysis of the antigen substance has not been possible.

Thus Spiro has reported that two glycopeptides can be isolated from bovine GBM (Spiro, R.G., "J.Biol. Chem.", Vol. 242, No. 8, 1923 - 1932 (1967) ).

Spiro's procedure for isolating the two glycopeptides is as follows. Firstly, GBM is separated from

kidney according to Krakower and Greenspon's method (Krakower and Greenspon "Arch. Path" 51, 629 - 639, (1951) ). The separated GBM is dispersed in 0.1 M tris acetate buffer and digested with collagenase at a pH of 7.4 and a temperature of 37$^{O}$C. The supernatant liquid obtained from centrifuging the digestion solution is then conditioned at pH 7.8 and subsequently is digested with pronase at 37$^{O}$C. This digestion solution is centrifuged to collect the supernatant liquid, which is then fractionated with Sephadex G-25. As shown in Figure 1 of Spiro's paper, two fractions which have sugar peaks 1 and 2 (by anthrone) respectively are obtained by this fractionation. The fraction (sugar peak 1) belonging to the void volume of Sephadex G-25 is further fractionated with Sephadex G-50. As shown in Figure 5 of the paper, sugar peak 1 is divided into two sugar peaks (by anthrone), one of them belonging to the void volume of Sephadex G-50.

Spiro found from this procedure two glycopeptides from the sugar peak 2 in Figure 1 and the right sugar peak in Figure 5. The glycopeptide of the sugar peak 2 is disaccharide comprising glucose and galactose in the ratio of 1:1 and one molecule of hydroxylysin, while the glycopeptide of the other sugar peak is heteropolysaccharide comprising galactose, mannose, glucosamine and fucose. Neither of the two glycopeptides isolated was found to have biological activity.

Spiro discarded the left sugar peak in Figure 5 because it belonged to the void volume of the molecular sieve and thus, in accordance with common knowledge in this field, was simply an impurity.

In studies made to establish an experimental model for human glomerulonephritis, especially for adult human glomerulonephritis, it has now surprisingly been found that the left sugar peak in Figure 5 which had been discarded by Spiro contains a substance which has

an ability to induce glomerulonephritis in animals which substance is quite different from the two glycopeptides isolated by Spiro. It has also been found that the biological activity of this glycopeptide substance is displayed by injecting it directly in to an animal. Such biological activity has been termed "nephrotogenicity", as opposed to the biological activity in Masugi's glomerulonephritis which is termed "nephrotoxicity".

According to the present invention there is provided a nephrotogenic compound characterised in that it contains a biologically active sugar moiety having the structural formula

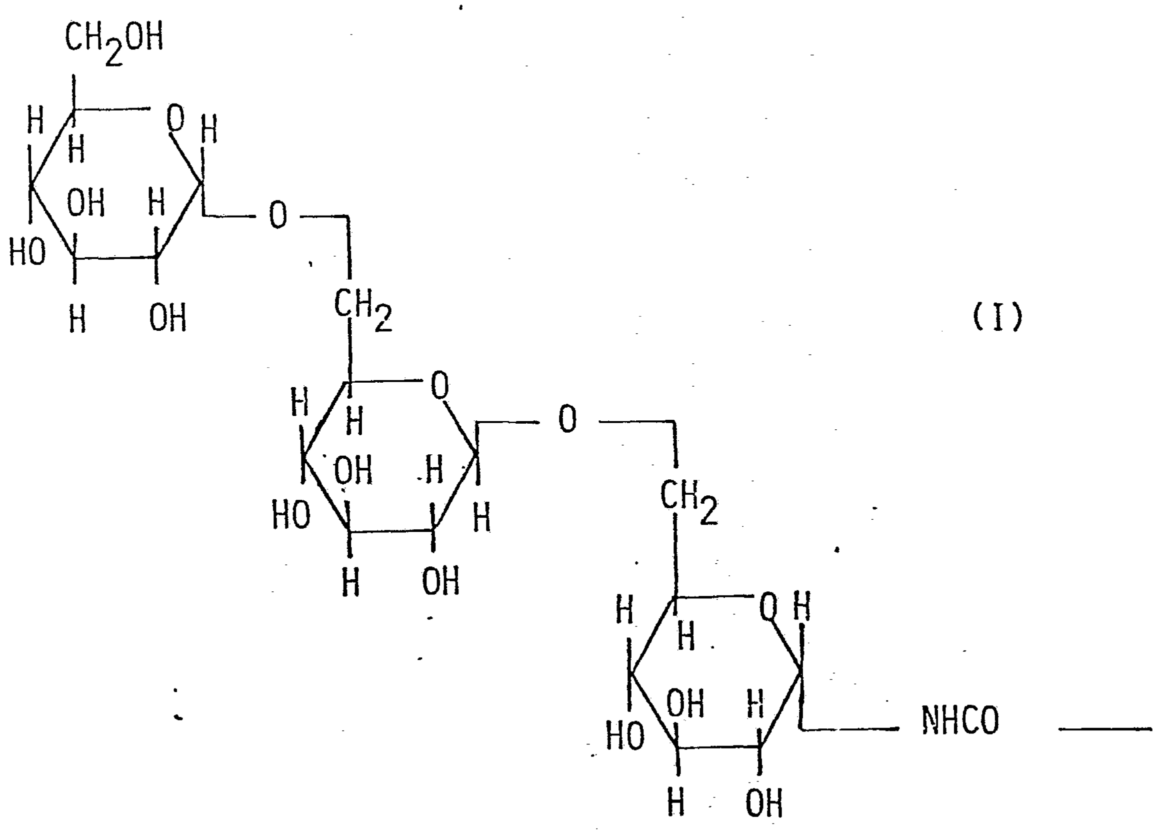

(I)

For example the compound may be a glycopeptide which contains a peptide moiety linking to the sugar moiety of formula (I) through the N-glycoside bond.

Another aspect of the invention provides a method of isolating the nephrotogenic compound defined above from materials which contain the compounds or their precursors in impure form which method is characterised by the steps of

(a) converting the impure compound or precursor to a form which is suceptible to digestion by means of a non-specific mixture of proteases;

(b) digesting the susceptible form produced in step (a) with a non specific mixture of proteases such as pronase and separating the digestion products;

(c) dialyzing the active product of step (b) to remove the dialyzable product and separate the non-dialyzable product therefrom; and

(d) treating the non-dialyzable product of step (c) with a protein precipitant such as trichloroacetic acid and separating the products of the treatment to form the desired nephrotogenic compound in substantially pure form.

In one embodiment the method is characterised in that the nephrotogenic compound is a glycopeptide consisting of a sugar moiety of structural formula (I) and a peptide moiety linking to the sugar moiety through the N-glycoside bond, and in that the material containing the impure compound or precursor is an organ or tissue of a normal animal, the method steps comprising

(1)digesting the organ or tissue with a protein-digesting enzyme and separating the digestion products by centrifuging;

(2) further digesting the supernatant liquid separated in step (1) with pronase and separating the further digestion products by centrifuging;

(3) dialyzing the supernatant liquid separated in step (2) to remove the dialyzate and separate the non-dialyzable product therefrom; and

(4) treating the non-dialyzable product of step (3) with trichloroacetic acid and separating the products of the treatment by centrifuging, contacting the supernatant liquid produced thereby with a lectin such as concanavalin A and eluting the pure nephrotogenic compound trapped thereon.

In another embodiment, step (4) is carried out by treating the non-dialyzable product of step (3) with trichloroacetic acid and separating the products of the treatment by centrifuging, contacting the supernatant liquid produced thereby with a non-water soluble material having negative charge and eluting the pure nephrotogenic compound adsorbed on the non-water soluble material.

The organ or tissue from which the glycopeptide is isolated may be for example kidney, lung, aorta, liver, spleen, heart or muscle.

In yet another embodiment the glycopeptide is isolated from urine from a healthy human being or a concentrate thereof, and the method steps comprise

(1) contacting the urine or urine concentrate with a non-water soluble material having negative charge and eluting the substance adsorbed thereon;

(2) digesting the eluate with pronase and separating the digestion products by centrifuging;

(3) dialyzing the supernatant liquid separated in step (2) to remove the dialyzate and separate the non-dialyzable product therefrom; and

(4) treating the non-dialyzable product of step (3) with trichloroacetic acid to yield the desired nephrotogenic compound in substantially pure form. If necessary, this method may include the additional step of contacting the product of step (4) with a lectin such as

concanavalin A and eluting the substance trapped thereon, further to purify the product.

The non-water soluble material having negative charge which is used in the above methods may be for example a polymeric material such as polyvinyl chloride, polyacrylonitrile, cellulose or cotton containing a functional group having negative charge such as nitrile, carboxyl, sulphonic or a halogen atom. Alternatively the material may be inorganic, such as porous glass, zeolite, silica gel or sellaite.

The nephrotogenic compounds of the invention, or agents which contain such compounds, e.g. the glycopeptide, as an active component together with a carrier, may be used as substances for inducing glomerulonephritis in animals. Thus the invention also extends to a method of preparing an experimental model for the study of human glomerulonephritis which is characterised in that a compound or agent as defined above is injected into an experimental animal to induce lesions similar to those of human glomerulonephritis.

The compounds of this invention contain a sugar moiety of novel structural formula (I) which has been found to have a unique property. Thus, while Masugi's glomerulonephritis is induced through the antigen-antibody reaction, glomerulonephritis, in the case of the present invention, may be induced by a single injection in the hind footpad of the animal, and the induced glomerulonephritis makes progress to chronic glomerulonephritis (contracted kidney) some 6 to 8 months from the injection.

The nephritogenicity of the compounds, e.g. the glycopeptide, is derived from the sugar moiety. It has been confirmed that even if the peptide moiety is

destroyed by suitable means such as digestion with trypsin or collagenase and pronase, the nephritogenicity of the compound remains, whereas if the sugar moiety is destroyed by periodate ($IO_4$) oxidation, glomerulonephritis is not induced at all.

The invention is further discussed with reference to the accompanying drawings, by way of example only, in which:

Figure 1 is a gas-chromatographic chart of a product obtained by methylation of a glycopeptide in accordance with the present invention;

Figure 2 is a $^{13}C$ NMR spectrum of the glycopeptide of Figure 1;

Figure 3 is a microscopic photograph showing renal tissue of a rat 8 months after an injection of the glycopeptide; and

Figure 4 is a microscopic photograph showing renal tissue of a normal rat.

The determination of structure of the sugar moiety which controls the nephritogenicity will be detailed heinafter.

i. The sugar consists of glucose only

According to the usual method for the determination of sugar structure (Hakomori, S.J. Biochem. 55, 205 - 208, (1964) ), the glycopeptide sample isolated from rat GBM was methylated and the methylated product was subjected to gas-chromatography. Figure 1 shows the gas-chromatographic spectrum which was obtained. The compounds in the peaks of the spectrum were identified by mass spectrometry, the results being given in the following Table.

Table

| peak | compound |
|---|---|
| A | 2,3,4,6-tetra-O-methyl-D-glucose |
| B | 3,4,6-tri-o-methyl-D-glucose |
| C | 2,3,6- and 2,3,4-tri-O-methyl-D-glucose |
| D | 3,6-di-O-methyl-D-glucose |
| E | 2,4-di-O-methyl-D-glucose |

It was thus found that the sugar moiety is composed of glucose residues only.

ii. Non-reducing terminus of sugar moiety is α-D-glucose

It was found that the glycopeptide of the present invention has a characteristic of bonding to concanavalin A (Con A). It is well known that Con A has a property of reacting selectively with polysaccharide which has any of α-D-glucose, α-D-mannose, or β-D-fructofuranosyl unit in its non-reducing terminus so as to produce precipitates. In view of this fact and the results of determination i, it was decided that the glucose in the non-reducing terminus of the sugar moiety is α-D-type.

iii. sugar moiety consists of three glucose residues

The glycopeptide was analysed by $^{13}C$ NMR spectrometry using t-butyl alcohol as internal standard (solvent : $D_2O$). Figure 2 shows the $^{13}C$ NMR spectrum thus obtained.

According to J.B. STOTHERS, "Carbon-13 NMR Spectroscopy" Academic Press, New York, (1972), Champ. 11 and E. BREITMAIER and W. VOELTER, "$^{13}C$ NMR Spectroscopy", Methods and Applications, Verlag Chemic Weinheim, Germany, (1974), Chap. 5, it is well known that the

chemical shift range of carbon C(1) in glucose and its analogues, which is bonded to two oxygen atoms, is 110 - 90 PPM (PPM from TMS) and that of carbons C(2) - C(6) which are bonded to one oxygen atom, is 85 - 60 PPM.

It is evident from the studies of the intensities of the signals in the region of 110 - 60 PPM that the number of carbons in the sugar moiety is less than 24. This indicates that the number of sugar residues in the sugar moiety is 3 or 4, because one glucose ring has six carbon atoms and there is some possibility that the signals of other carbons in the peptide moiety of the glycopeptide are in this region.

The glycopeptide has only two peaks A (104.2 PPM) and B (100.2 PPM) in the region of the chemical shifts of carbon (1) in the glucose ring. The intensities of these two signals are almost identical. This fact eliminates the possibility of the presence of four glucose residues in the glycopeptide. Accordingly, it is evident that the sugar moiety is composed of three glucose residues.

iv. sugar moiety has a bond pattern of

$$glu \xrightarrow{\alpha-1.6} glu \xrightarrow{\beta-1.6} glu \xrightarrow{\alpha}$$

In order to assign the signals of carbons in the sugar moiety of the glycopeptide, $^{13}C$ NMR spectra of 27 kinds of analogous sugars and their derivatives were measured at almost the same condition. From the analyses of these $^{13}C$ NMR spectra, it has become evident that the signal B (100.2 PPM) (see Figure 2) can be assigned to the carbon (1) of α-1,6 glucose-glucose linkage, because the observed chemical shift of isomaltose ( α-1,6 linkage) (100.5 PPM) is very close to that of B. However, the signal A (104.2 PPM) cannot be immediately assigned, because any peaks of analogous sugars are not so close to that of A. As the chemical shifts of one glucose ring are influenced by the type of linkage of the second and/or the third glucose ring, substituent

effects by the glucose ring on the chemical shifts were obtained by detailed analysis of the chemical shifts of glucose-glucose chain compounds with α-, β-1,1-, 1,2-, 1,3-, 1,4-, and 1,6-linkages. Using the values of the substituent effects and the chemical shifts of glucose thus obtained, the expected chemical shifts of many types of combinations of three glucose rings were calculated. Thus, the following five types were chosen as the appropriate combinations to fit the two peaks A and B the glycopeptide from the analyses of C(1) chemical shifts.

I. Glc $\xrightarrow{\alpha-1,6}$ Glc $\xrightarrow{\beta-1,6}$ Glc $\overset{\alpha}{—}$ P

II. Glc $\xrightarrow{\beta-1,6}$ Glc $\xrightarrow{\alpha-1,6}$ Glc $\overset{\alpha}{—}$ P

III. Glc $\xrightarrow{\beta-1,6}$ Glc $\xrightarrow{\alpha-1,4}$ Glc $\overset{\alpha}{—}$ P

IV. Glc $\xrightarrow{\beta-1,4}$ Glc $\xrightarrow{\alpha-1,6}$ Glc $\overset{\alpha}{—}$ P

V. 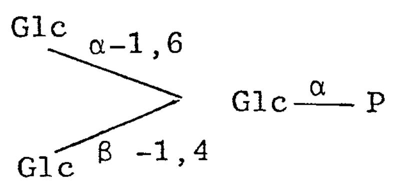

In the analyses of the chemical shift range of 85 - 60 PPM, it is expected that the chemical shift of the carbon bonded at the 4-position of glucose of β-1,4 linkage must be near 81.4 - 81.2, considering the experimentally obtained value of cellobiose (β -1,4 linkage) (81.2 PPM) and the results of the calculations. However, such a signal cannot be found in the spectrum of the glycopeptide. The chemical shift of its nearest peak is 77.7 PPM, but this is too remote. Therefore, types III, IV and V are excluded.

Moreover, from the Concanavalin A test, it has been made clear that the non-reducing terminus glucose in the glycopeptide has an α-configuration. This and the $^{13}C$ NMR analyses therefore indicate that the sugar moiety has an α-1,6-β-1,6-α chain configuration.

v. peptide moiety links to sugar moiety through N-glycoside bond

The glycopeptide of the present invention was hydrolysed according to the method of hydrolysis with strong alkali which is usually used for destroying the N-glycoside bond between asparagine or glutamine and sugar (Lee , Y.C. and Scocca, J.R. "J.Biol. Chem." 247, 5753 -5758, (1972) ).. Most of the products were trisaccharide, corresponding with the results of determination iii).

Moreover, with respect to $^{13}C$ NMR, a methine carbon which is bonded to one oxygen atom and one nitrogen atom (-O-CH-N<) is expected to resonate at a much higher field (probably in the range of 85 - 70 PPM) than that bonded to two oxygen atoms. Taking this and only two signals with the same intensities for C(1) into account, it can be said that the sugar moiety has an N-glycoside linkage at C(1) of the terminal glucose, instead of the peptide linkage through a glucosamine.

From these facts, it is clear that the sugar moiety links to the peptide moiety through N-glycoside bond.

In view of the facts mentioned in determinations i - v, it was concluded that the sugar moety has structural formula (I).

A general example of the method of isolating the glycopeptide of the invention is detailed hereinafter.

The GBM prepared according to Krakower and Greenspon's method was digested with a protein-digesting enzyme such as trypsin or collagenase and centrifuged to yield a supernatant liquid which was further digested with pronase, and centrifuged. The supernatant liquid thereby obtained was dialyzed to remove dialyzable disaccharide (corresponding to Spiro's

sugar peak 2), and the remaining non-dialyzable solution was lyophilized. The powder thus produced was dissolved in water and trichloroacetic acid was added to the solution to form precipitates which were removed by centrifuging. Since trichloroacetic acid has the characteristic of removing selectively glycopeptide containing mannose as sugar component, as well as the well known characteristic of reacting with free protein to form precipitates, Spiro's heteropolysaccharide (corresponding to the right sugar peak in Figure 5 mentioned above) was removed. Thereafter, the supernatant liquid was subjected to Con A affinity chromatography. As mentioned above, Con A has the characteristic of bonding to α-D-glucose in the non-reducing terminus of polysaccharide. Accordingly, the glycopeptide of the present invention was trapped on the Con A; this was subsequently eluted with α-D-methyl mannose.

It has been found that the glycopeptide is adsorbed selectively on non-water soluble materials having negative charge, and so it is possible in the method to utilize a step of contacting the supernatant liquid with such a non-water soluble material and eluting the substance adsorbed on the non-water soluble material, instead of the step of Con A affinity chromatography.

As the non-water soluble material having negative charge, there may be used, for example polymeric materials containing a functional group having negative charge or inorganic materials having negative charge. Typical examples of the former are the materials containing nitrile group, carboxyl group, sulphonic acid group or halogen atom, preferably polyvinyl chloride, polyacrylonitrile, cellulose or cotton. Typical examples of the latter are porous glass, zeolite, silica gel, and sellaite.

The optimum conditions for the adsorption step depend on the type of non-water soluble material which is used. It is preferred to carry out the step at a pH ranging from neutral to slightly acidic.

As eluting solvent, it is possible to use weakly alkaline aqueous solutions such as dilute aqueous ammonia or aqueous solutions of electrolytes such as sodium chloride or agents for modifying proteins such as urea.

Since the glycopeptide of the invention is present in organs or tissue other than kidney, such as lung, aorta, liver, spleen, heart and muscle, it is possible to use these as a starting material.

Further since the glycopeptide is present in human urine, it is possible to isolate the glycopeptide starting from this source, preferably from the urine of healthy human males. In this case, the urine is first contacted with non-water soluble material and the adsorbed substance is eluted after washing of the non-water soluble material with distilled water. It is possible to carry out the adsorption with good efficiency by first concentrating the urine according to the usual methods such as concentration under reduced pressure, concentration with ultrafiltration or foaming concentration. It is preferred to regulate the urine at a pH ranging from neutral to slightly acidic. In this case, it is also possible to use as eluting solvent a weakly alkaline aqueous solution, or an aqueous solution containing electrolyte or an agent for modifying protein.

The thus obtained eluate is digested with pronase and is centrifuged to yield a supernatant liquid which is then dialyzed. The non-dialyzable

substance remaining is treated with trichloroacetic acid and the precipitates produced are removed by centrifuging. If necessary, the obtained supernatant liquid is further subjected to Con A affinity chromatography and the substance trapped on the Con A is eluted with an eluent.

The nepritogenicity of the glycopeptide will be described hereinafter.

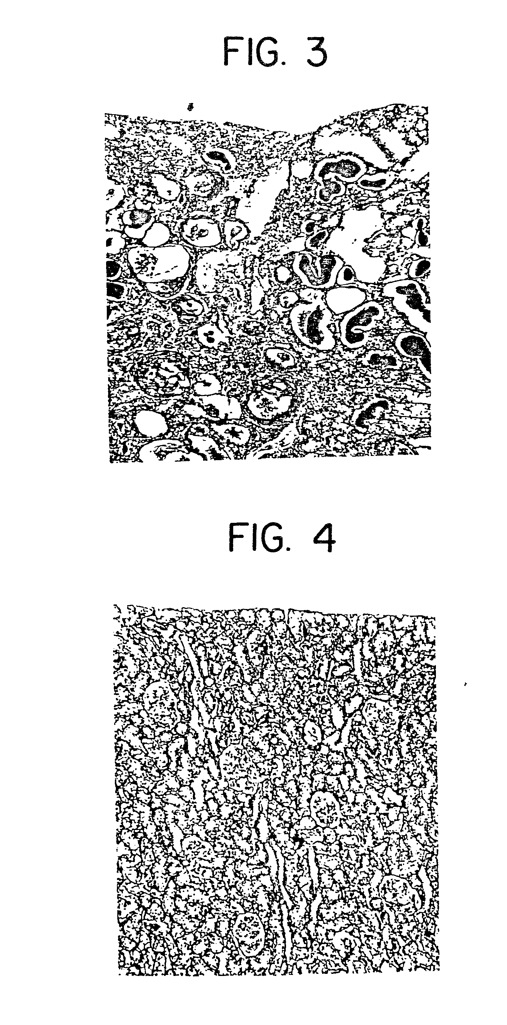

In animals which received a single footpad injection of the glycopeptide with Freund's incomplete adjuvant, proteinuria began to appear 3 to 4 weeks after the injection and increased gradually. Morphologic changes of proliferative glomerulonephritis appeared 1.5 months after the injection and progessed, 6 to 8 months after injection, to typical chronic glomerulonephritis (the contracted kidney). Figure 3 shows the lesional kidney 8 months after the injection. It is found from the comparison with normal kidney (shown in Figure 4) that the morphologic changes in the kidney have progressed to typical chronic glomerulonephritis.

Furthermore, as the result of disorders of clinical metabolism, unique uremic osteodystrophy (a bone disease which is a secondary symptom of chronic renal failure) was produced in animals: that is, severe osteodystrophy was induced, 300 to 400 days after the footpad injection in these animals, with marked (over 10 fold) hyperplasia of the parathyroid gland and the decrease of serum Ca level.

The reproducing efficiency of nephritogenicity produced by the glycopeptide was found to be almost 100 %.

The following non-limiting examples illustrate the invention. Some of the examples relate to the isolation of compounds from organs excised from rat.

It is possible, of course, to use as starting material organs excised from animals other than rat, such as, bovine animals, dogs, rabbits and mice.

Example 1: Isolation from rat GBM

Kidney excised from 1200 rats was washed fully with physiological salt solution under reflux to remove blood and thereafter was cut finely to collect renal cortex (Yield: about 1 kg (wet weight) ).

The GBM was prepared from the renal cortex according to Krakower and Greenspon's method. More detailedly, the renal cortex was cut finely and separated with sieves having 150 mesh and 100 mesh and finally 170 mesh. The glomerulus remaining on the sieve having 170 mesh was washed fully with physiological salt solution and was subjected to treatment with supersonic waves. After washing with physiological salt solution many times and then with distilled water, the solution containing destroyed glomerulus was centrifuged at low speed. Pure GBM 20 g (wet weight) was obtained as precipitate.

The GBM was dissolved in a small amount of physiological salt solution adjusted to pH 8.0 with 0.1 M sodium borate. Trypsin was added in the amount of 0.5 % of the weight of GBM and the mixture was incubated at 37°C for 3 hours. The mixture was heated at 60°C for 30 min. to inactivate the trypsin and was centrifuged at 27,000 rpm for 35 min. The supernatant liquid was then lyophilized to powder form.

The powder was then dissolved in a small amount of physiological salt solution and the mixture was adjusted to pH 7.8 with tris acetate buffer and digested with pronase. This enzyme was added initially in the amount of 0.3% with further enzyme additions of 0.1% at 24 and 48 hours. This incubation was carried out for a

total period of 72 hours at 37°C. Thereafter, the undigested material was removed by centrifuging and the supernatant liquid obtained was lyophilized to powder form.

The powder was dissolved in distilled water to form a solution which was charged into a cellulose tube (Visking tube, 27/32 inch), and the cellulose tube was dipped into flowing water. Dialysis was continued for 3 days. In this way, dialyzable disaccharide was removed.

Trichloroacetic acid was then added to the non-dialyzable materials in the amount of 5% and the mixture was stood still for a short time. The precipitates which formed were removed by centrifuging at 3,000 rpm for 30 min., whereby heteropolysaccharide was removed.

The obtained supernatant liquid was fed to a column filled with Con A. After washing the column with distilled water, the substance trapped on the Con A was eluted with α-D-methyl mannose. The eluate was lyophilized so as to obtain the glycopeptide (10 mg).

It was confirmed by $^{13}C$ NMR analysis that the glycopeptide obtained by the process mentioned above has two signals at 104.2 PPM and 100.2 PPM.

Example 2: Isolation from rat renal cortex

The renal cortex (about 1 kg wet weight), treated similarly to Example 1, was separated with a 9 mesh sieve. The renal cortex which passed through the sieve was washed with small amount of physiological salt solution and then was dialyzed and lyophilized after desalting. The powder was dissolved in water and adjusted to pH 8.0 with 0.1 M sodium borate.

The preparation of the glycopeptide was carried out using this solution as a starting material similarly to Example 1. The glycopeptide was obtained in a yield of 6 to 8 mg.

Example 3: Isolation from rat lung

The lung excised from rat was washed fully with physiological salt solution under reflux in order to remove blood. The lung was cut finely and put into acetone, and the tissue which precipitated was collected and dried after removing the acetone. Then, the powder was separated with a 9 mesh sieve to remove bronchus. The thus obtained lung tissue (200 g dry weight) was used as a starting material in a preparation which was carried out similarly to Example 1. The glycopeptide was obtained in a yield of 10 mg.

Example 4: Isolation from rat aorta

The aorta excised from rat was put into acetone. After some days, acetone was removed and the aorta was dried and milled in a mortar. The thus obtained aorta tissue (120 g) was used as a starting material for the preparation of glycopeptide which was carried out similarly to Example 1. The glycopeptide was obtained in a yield of 10 mg.

Example 5: Isolation from rat liver

The liver excised from rat was washed fully with physiological salt solution under reflux to remove blood and then homogenized. The homogenate was then centrifuged at 8,000 rpm to collect precipitates.

The isolation of glycopeptide was carried out using the precipiates (1,000 g wet weight) similarly to Example 1. The glycopeptide was obtained in a yield of 5 mg.

Example 6: Isolation from rat spleen

Using spleen excised from rat, the starting material was prepared similarly to Example 5.

The isolation of glycopeptide from the starting material (1,000 g wet weight) was carried out similarly

to Example 1. The glycopeptide was obtained in a yield of 2 to 3 mg.

Example 7: Isolation from rat heart

In this case, the homogenate prepared by homogenizing heart excised from rat was used as a starting material.

Using the homogenate (1,000 g) the isolation of glycopeptide was carried out similarly to Example 1. The glycopeptide was obtained in a yield of 6 mg.

Example 8: Isolation from rat muscle

Using rat muscle, the starting material was prepared similarly to Example 5.

The isolation of glycopeptide from this starting material (1 kg) was carried out similarly to Example 1. The glycopeptide was obtained in a yield of 2 mg.

Example 9

The preparation of glycopeptide from rat GBM was carried out in accordance with Example 1 but, instead of the step of Con A affinity chromatography, the step of adsorption with non-water soluble material was used in order to extract selectively the glycopeptide. More detailedly, the supernatant liquid obtained after the treatment with trichloroacetic acid was fed to a column filled with sellaite. After washing the sellaite with distilled water, the glycopeptide adsorbed on the sellaite was eluted with aqueous ammonia solution. The eluate was neutralized and lyophilized after desalting; the glycopeptide was obtained in a yield of 10 mg.

It was confirmed by $^{13}C$ NMR analysis that the

glycopeptide obtained had two signals at 104 PPM and 100 PPM.

Similar results were obtained when polyacrylonitrile fibre, polyvinyl chloride, cellulose, cotton, porous glass, zeolite, and silica gel was used instead of sellaite.

Example 10: Isolation from human urine

Urine (1,000 l) from healthy males was concentrated by feeding air through it for 2 hours and then adding a small amount of octanol to the foam which was produced. In this way, concentrated urine (100 l) was obtained. The concentrated urine was diluted with distilled water (100 l) and adjusted to pH 6.5. To this solution, sellaite (100 l) was added and the mixture was agitated for 1 hour. Thereafter, the sellaite was collected by filtration and the separated sellaite was washed with alkaline water. The substance adsorbed on the sellaite was then eluted with alkaline solution containing sodium chloride. The eluate was neutralized and lyophilized after desalting with superfiltration and concentration. Dry urine powder (2 g) was obtained.

The powder was dissolved in a small amount of physiological salt solution and the mixture was adjusted to pH 7.8 with Tris acetate buffer and digested with pronase. This enzyme was added initially in the amount of 0.3% and further in the amount of 0.1% at 24 and 48 hours. The incubation was carried out for a total period of 72 hours at 37$^{o}$C. Thereafter, the undigested material was removed by centrifuging and the supernatant liquid obtained was lyophilized to powder form.

The powder was dissolved in distilled water and the solution produced was charged into a cellulose tube (Visking tube, 27/32 inch) which was dipped into flowing water. Dialysis was continued for 3 days so as to remove dialyzable disaccharide.

Trichloroacetic acid was added to the remaining non-dialyzable materials in an amount of 5% and the mixture was stood still for short time. The precipitates produced were removed by centrifuging at 3,000 rpm for 30 min., and the supernatant liquid obtained was lyophilized (yield of 10 - 15 mg).

In order to obtain the glycopeptide having higher purity, the supernatant liquid produced after the treatment with trichloroacetic acid was fed to a column filled with Con A. After the column was washed with distilled water, the substance trapped on the Con A was eluted with α-D-methyl mannose and the eluate was lyophilized.

It was confirmed by $^{13}C$ NMR analysis that the glycopeptide from human urine also had two signals at 104 PPM and 100 PPM.

Example 11

The concentrated urine (100 l) obtained by a treatment similar to that of Example 10 was adjusted to pH 5.5. and then fed gradually to a column filled with polyacrylonitrile fibre (300 g). After the polyacrylonitrile fibre was washed fully with water, the substance adsorbed thereon was eluted with 4% aqueous ammonia solution. The eluate was neutralised and treated similarly to Example 10 so as to obtain dry urine powder (2 g).

The isolation of glycopeptide from the urine powder was carried out similarly to Example 10. The glycopeptide was obtained in a yield of 10 - 15 mg.

Example 12

Using polyvinyl chloride instead of polyacrylonitrile fibre, dry urine powder (2 g) was obtained similarly to Example 11.

The isolation of glycopeptide from the dry urine powder was carried out similarly to Example 10 (Yield: 10 - 15 mg).

Example 13

Human urine (1,000 l) was adjusted to pH 7.5. After the produced precipitates had been removed, the urine was fed at a rate of 500 cc/min. to a column filled with silica gel (30 l) which had previously been washed with 5% HCl and 10% NaCl aqueous solution. Thereafter, the column was washed with water and the substance adsorbed on the silica gel was eluted with 4% aqueous ammonium solution (4 l). The eluate was neutralized with HCl, concentrated, and finally lyophilized so as to obtain dry urine powder (4 g).

The isolation of glycopeptide from the dry urine powder was carried out similarly to Example 10 (Yield: 20 - 25 mg).

Example 14

Human urine (1,000 l) was fed at a rate of 200 l/hour to a column (diameter 6 cm; height 30 cm) filled with porous glass (1 l) having a mesh size of 120 - 200 mesh. Thereafter, the column was washed with water (10 l) and the substance adsorbed on the porous glass was eluted with 4% aqueous ammonia solution (4 l). The eluate was neutralized with 5% HCl, concentrated, and finally lyophilized so as to obtain dry urine powder (4.5 g).

The isolation of glycopeptide from the dry urine powder was carried out similarly to Example 10 (Yield: 20 - 25 mg).

The nephritogenicity of glycopeptide obtained in the Examples mentioned above is illustrated by the following experiments.

Experiment 1

The glycopeptide isolated from rat GBM (Example 1) was injected in to the footpad of rat with Freund's incomplete adjuvant. The amount of glycopeptide injected was 300 - 500 μg. 8 Months after the injection the rat was killed and dissected. It was confirmed that morphologic changes of kidney progressed to the contracted kidney which is a typical change of chronic glomerulonephritis.

Experiment 2

The glycopeptide (300 - 500 μg) isolated from rat lung (Example 3) was injected in to the footpad of rat. 8 Months after the injection, it was confirmed that morphologic changes of the kidney had progressed to the contracted kidney stage.

When the glycopeptide isolated from other organs, that is, aorta, liver, spleen, heart or muscle was injected in to rat, the morphologic changes observed were the same as those observed in the case of GBM or lung - derived glycopeptide.

Experiment 3

The glycopeptide (300 - 500 μg) isolated from human urine (Example 10) was injected in to the footpad of rat. 8 Months after the injection, it was observed that morphologic changes of kidney had progressed to the contracted kidney stage.

Experiment 4

The glycopeptide (300 - 500 μg) isolated from rat was injected in to dog with Freund's incomplete adjuvant. Similarly to the case of rat, 8 months after the injection, the morphologic changes of the kidney progessed to the contracted kidney stage.

CLAIMS:

1. A nephrotogenic compound characterised in that it contains a biologically active sugar moiety having the structural formula

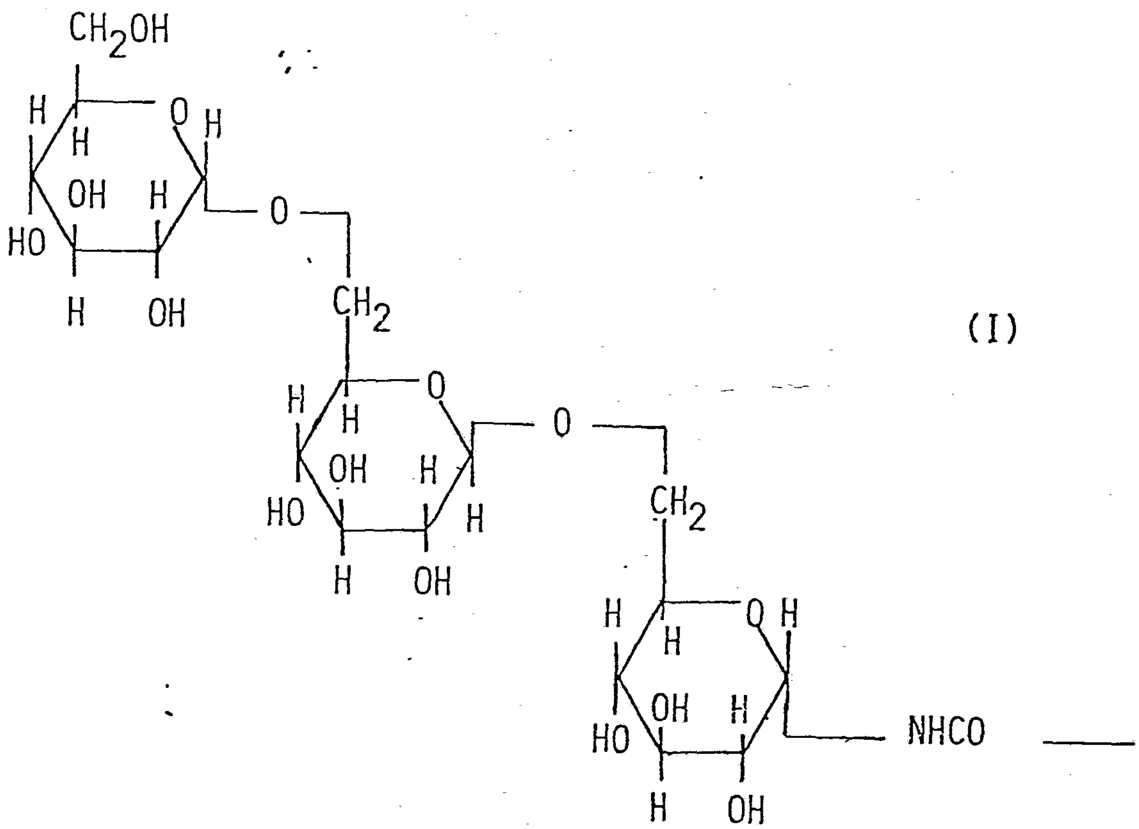

(I)

2. A compound as claimed in Claim 1 characterised in that it is a glycopeptide which contains a peptide moiety linking to the sugar moiety through the N-glycoside bond.

3. An agent for inducing glomerulonephritis in an animal characterised in that it comprises a nephrotogenic compound as claimed in claim 1 or 2 as active component, and a carrier therefor.

4. A method of preparing an experimental model for the study of human glomerulonephritis characterised in that a nephrotogenic compound as claimed in claim 1 or 2, or an agent as claimed in claim 3, is injected into an experimental animal to induce lesions similar to those of human glomerulonephritis.

5. The use of a nephrotogenic compound as claimed in claim 1 or 2, or of an agent as claimed in claim 3, as a substance for inducing glomerulonephritis in an animal.

6. A method of isolating a nephrotogenic compound containing a biologically active sugar moiety having the structural formula (I) as set out in claim 1 from a material containing the compound or a precursor thereof in impure form, which method is characterised by the steps of

(a) converting the impure compound or precursor to a form which is susceptible to digestion by means of a non-specific mixture of proteases;

(b) digesting the susceptible form produced in step (a) with a non specific mixture of proteases such as pronase and separating the digestion products;

(c) dialyzing the active product of step (b) to remove the dialyzable product and separate the non-dialysable product therefrom; and

(d) treating the non-dialyzable product of step (c) with a protein precipitant such as trichloroacetic acid and separating the products of the treatment to form the desired nephrotogenic compound in substantially pure form.

7. A method as claimed in claim 6 characterised in that the nephrotogenic compound is a glycopeptide consisting of a sugar moiety of structural formula (I) and a peptide moiety linking to the sugar moiety through the N-glycoside bond, and in that the material containing the impure compound or precursor is an organ or tissue of a normal animal, the method steps comprising

(1) digesting the organ or tissue with a protein-digesting enzyme and separating the digestion products by centrifuging;

(2) further digesting the supernatant liquid separated in step (1) with pronase and separating the further digestion products by centrifuging;

(3) dialyzing the supernatant liquid separated in step (2) to remove the dialyzate and separate the non-diayzable product therefrom; and

(4) treating the non-dialyzable product of step (3) with trichloroacetic acid and separating the products of the treatment by centrifuging, contacting the supernatant liquid produced thereby with a lectin such as concanavalin A and and eluting the pure nephrotogenic compound trapped thereon.

8. A method as claimed in claim 6 characterised in that the nephrotogenic compound is a glycopeptide consisting of a sugar moiety of structural formula (I) and a peptide moiety linking to the sugar moiety through the N-glycoside bond, and in that the material containing the impure compound or precursor is an organ or tissue of a normal animal, the method steps comprising

(1) digesting the organ or tissue with a protein-digesting enzyme and separating the digestion products by centrifuging;

(2) further digesting the supernatant liquid separated in step (1) with pronase and separating the further digestion products by centrifuging;

(3) dialyzing the supernatant liquid separated in step (2) to remove the dialyzate and separate the non-dialyzable product therefrom; and

(4) treating the non-dialyzable product of step (3) with trichloroacetic acid and separating the products of the treatment by centrifuging, contacting the supernatant liquid produced thereby with a non-water soluble material having negative charge and eluting the pure nephrotogenic compound adsorbed on the non-water soluble material.

9. A method as claimed in claim 7 or 8 characterised in that the organ or tissue is kidney, lung, aorta,

liver, spleen, heart or muscle.

10. A method as claimed in claim 6 characterised in that the nephrotogenic compound is a glycopeptide consisting of a sugar moiety of structural formula (I) and a peptide moiety linking to the sugar moiety through the N-glycoside bond, and in that the material containing the impure compound or precursor is urine from a healthy human being or a concentrate thereof, the method steps comprising

(1) contacting the urine or urine concentrate with a non-water soluble material having negative charge and eluting the substance adsorbed thereon;

(2) digesting the eluate with pronase and separating the digestion products by centrifuging;

(3) dialyzing the supernatant liquid separated in step (2) to remove the dialyzate and separate the non-dialyzable product therefrom; and

(4) treating the non-dialyzable product of step (3) with trichloroacetic acid to yield the desired nephrotogenic compound in substantially pure form.

11. A method as claimed in claim 10 characterised in that it includes the additional step of contacting the product of step (4) with a lectin such as concanavalin A and eluting the substance trapped thereon, further to purify the product.

12. A method as claimed in claim 8, 9, 10 or 11 characterised in that the non-water soluble material having negative charge is (i) a polymeric material such as polyvinyl chloride, polyacrylonitrile, cellulose or cotton containing a functional group or atom having negative charge such as a nitrile group, a carboxyl group, a sulphonic acid group or a halogen atom, or (ii) an inorganic material having negative charge such as porous glass, zeolite, silica gel or sellaite.

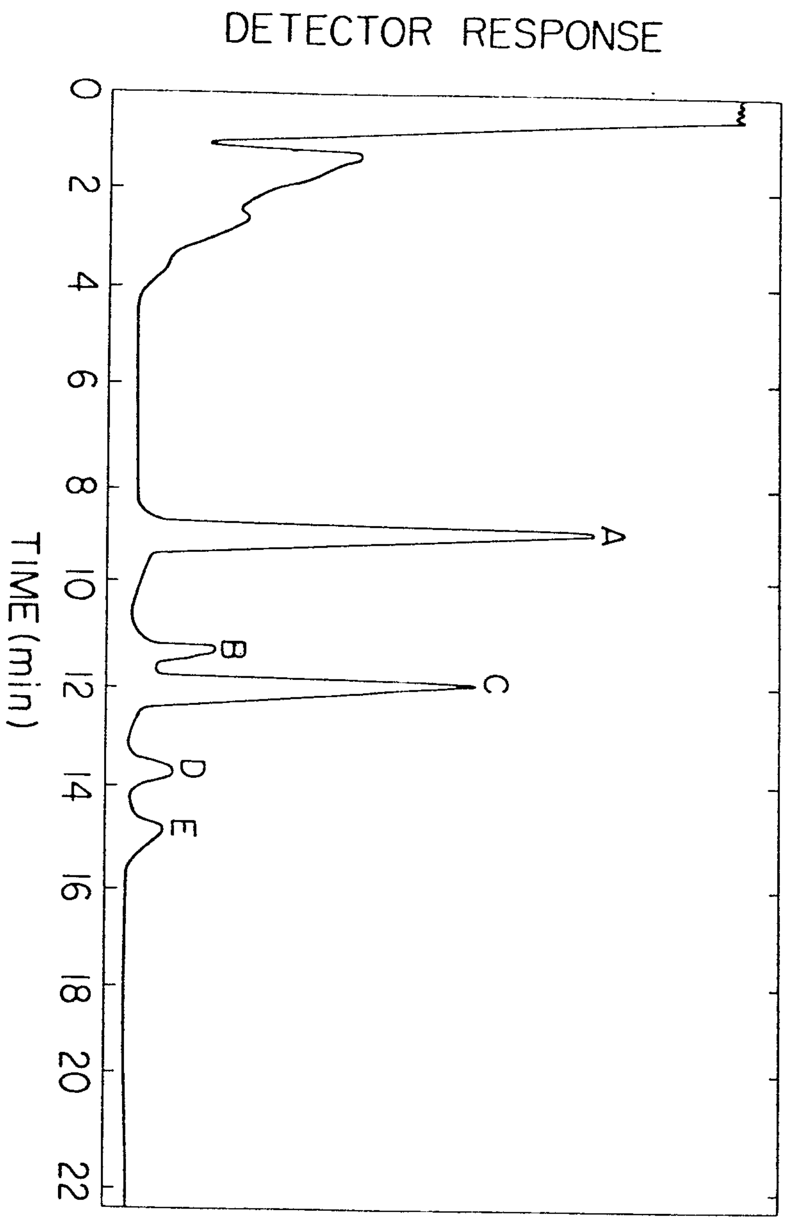


FIG. I

0026561



FIG. 2

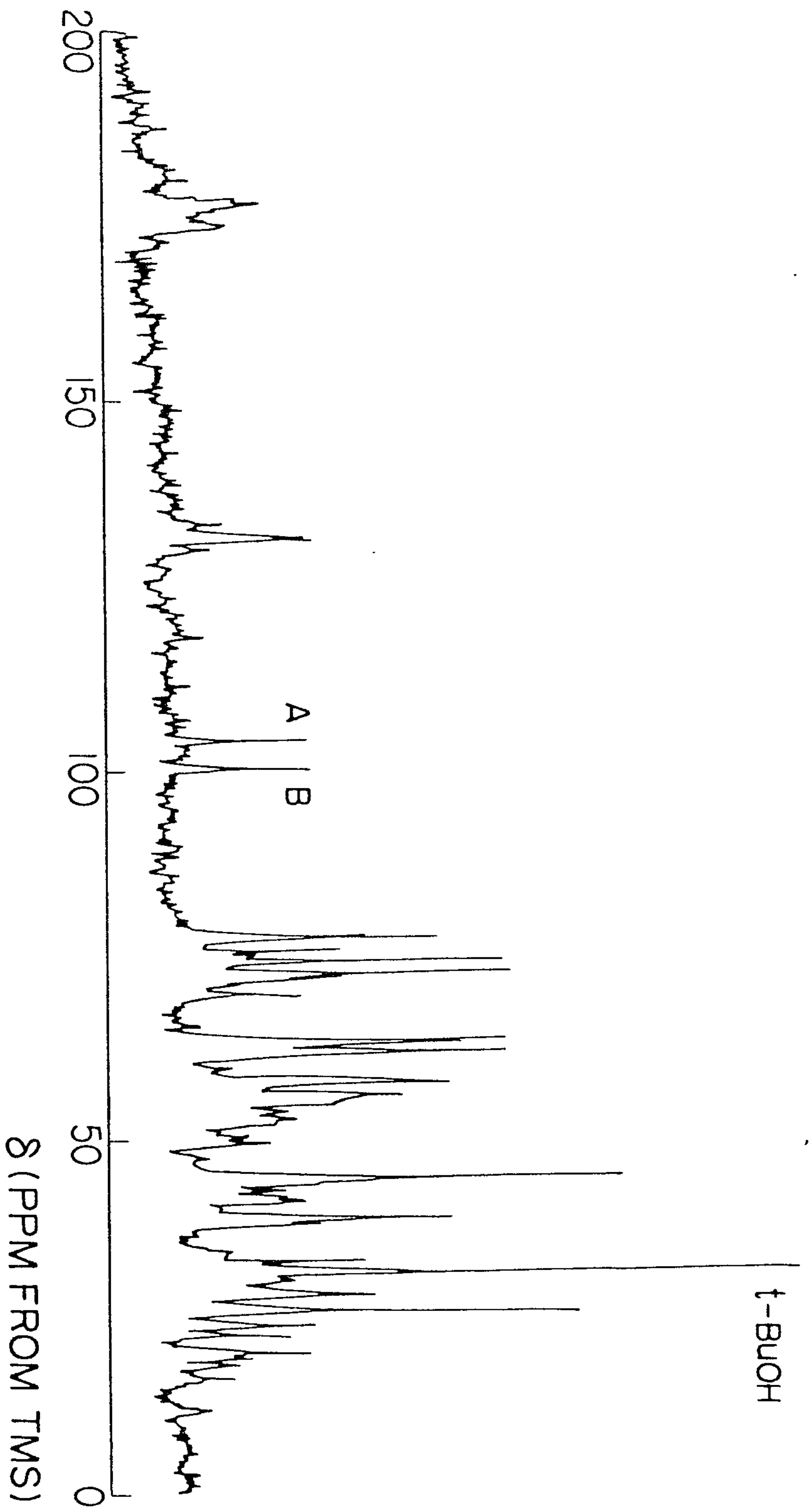

FIG. 3

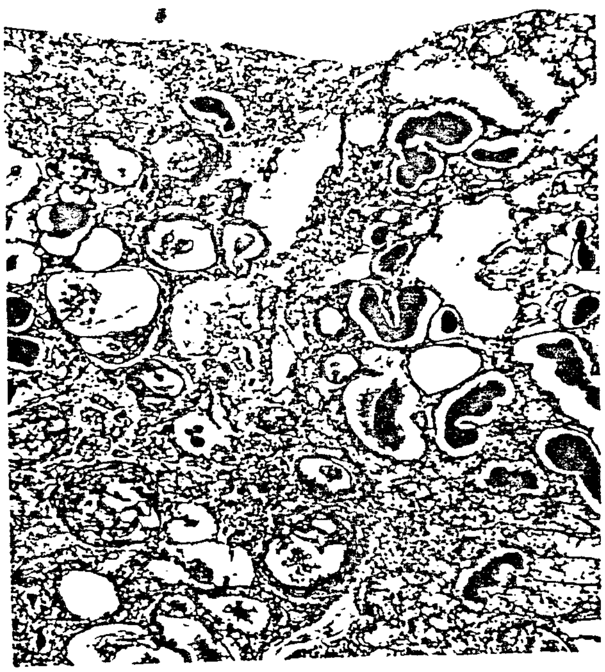

FIG. 4